# FASCICULE DE BREVET EUROPEEN

(11) **EP 2 392 669 B1**
(45) Date de publication et mention de la délivrance du brevet: **30.01.2013**
(21) Numéro de dépôt: 10306276.6
(22) Date de dépôt: 19.11.2010
(51) Int. Cl.: C12Q 1/68

(54) **Méthode de détection des leptospires pathogènes basée sur le gène rpoB**
Methode zum Nachweis von pathogenen Leptospiren, die auf dem rpoB-Gen basiert
Method for detecting pathogenic leptospires using the rpoB gene

(30) Priorité: 11.12.2009 FR 0958895
(43) Date de publication de la demande: 07.12.2011
(73) Titulaire: Institut d'Enseignement Supérieur et de Recherche en Alimentation, Santé Animale, Sciences Agronomiques et de l'Environnement, 69280 Marcy l'Etoile (FR)
(72) Inventeur: Kodjo, Angeli, 69210 Fleurieux sur l'Arbresle (FR); Djelouadji, Zoheira, 69002 Lyon (FR)
(74) Mandataire: Buchet, Anne

(56) Documents cités:
- FR-A1- 2 798 386
- LA SCOLA BERNARD ET AL: "Partial rpoB gene sequencing for identification of Leptospira species.", FEMS MICROBIOLOGY LETTERS OCT 2006 LNKD- PUBMED:16978348, vol. 263, no. 2, octobre 2006 (2006-10), pages 142-147, XP002580052, ISSN: 0378-1097
- RENESTO PATRICIA ET AL: "rpoB gene analysis as a novel strategy for identification of spirochetes from the genera Borrelia, Treponema, and Leptospira", JOURNAL OF CLINICAL MICROBIOLOGY, AMERICAN SOCIETY FOR MICROBIOLOGY, WASHINGTON, DC, US, vol. 38, no. 6, 1 juin 2000 (2000-06-01), pages 2200-2203, XP002155811, ISSN: 0095-1137
- CERQUEIRA GUSTAVO M ET AL: "Distribution of the leptospiral immunoglobulin-like (lig) genes in pathogenic Leptospira species and application of ligB to typing leptospiral isolates", JOURNAL OF MEDICAL MICROBIOLOGY, vol. 58, no. 9, septembre 2009 (2009-09), pages 1173-1181, XP002580053, ISSN: 0022-2615
- ADEKAMBI T ET AL: "The rpoB gene as a tool for clinical microbiologists", TRENDS IN MICROBIOLOGY, ELSEVIER SCIENCE LTD., KIDLINGTON, GB LNKD- DOI:10.1016/J.TIM.2008.09.008, vol. 17, no. 1, 1 janvier 2009 (2009-01-01), pages 37-45, XP025770209, ISSN: 0966-842X [extrait le 2009-01-01]
- SMYTHE LEE D ET AL: "A quantitative PCR (TaqMan) assay for pathogenic Leptospira spp", BMC INFECTIOUS DISEASES, BIOMED CENTRAL, LONDON, GB LNKD- DOI:10.1186/1471-2334-2-13, vol. 2, no. 1, 8 juillet 2002 (2002-07-08) , page 13, XP021015585, ISSN: 1471-2334
- STODDARD R A ET AL: "Detection of pathogenic Leptospira spp. through TaqMan polymerase chain reaction targeting the LipL32 gene", DIAGNOSTIC MICROBIOLOGY AND INFECTIOUS DISEASES, ELSEVIER SCIENCE PUBLISHING CO., AMSTERDAM, NL LNKD- DOI:10.1016/J.DIAGMICROBIO.2009.03.014, vol. 64, no. 3, 1 juillet 2009 (2009-07-01), pages 247-255, XP026152477, ISSN: 0732-8893 [extrait le 2009-04-22]

## Description

### DOMAINE TECHNIQUE

La présente invention s'inscrit dans la recherche de tests simples permettant le diagnostic des leptospires, dont les souches pathogènes provoquent la leptospirose.

Il est proposé d'utiliser le gène *rpoB,* et notamment une région de celui-ci, pour conclure à la présence d'une souche pathogène de leptospire.

### ETAT ANTERIEUR DE LA TECHNIQUE

Les leptospires pathogènes sont responsables de la leptospirose, zoonose émergente de répartition mondiale induite par des bactéries du genre *Leptospira.* Ces bactéries sont des spirochètes de forme hélicoïdale, extrêmement mobiles, essentiellement rencontrées en zones humides où elles sont entretenues par les rongeurs qui constituent leurs principaux réservoirs.

L'épidémiologie de cette zoonose est complexe, toutefois l'élimination rénale des leptospires par les animaux infectés, via leurs urines, est un élément clé pour leur persistance dans l'environnement. Une fois éliminées dans le milieu extérieur, les leptospires contaminent les sols et l'eau qui représentent alors une importante source de transmission indirecte de la maladie aux humains et aux animaux domestiques et sauvages, ce qui rend difficile la lutte contre ces bactéries.

Selon l'organisation mondiale de la santé, un demi-million de personnes par an sont diagnostiquées comme ayant été infectées par la leptospirose, principalement dans les zones tropicales et subtropicales mais aussi dans les zones tempérées. Dans les pays occidentaux, le risque sanitaire est lié essentiellement à la pratique d'activités de loisirs en lien avec l'eau.

Le diagnostic clinique de la leptospirose est complexe du fait de la diversité des symptômes liés à la fois aux souches infectantes, mais aussi aux espèces infectées. En effet selon la classification sérologique, le genre *Leptospira* est constitué d'espèces saprophytes (*Leptospira biflexa*) et pathogènes (*Leptospira interrogans*), divisé en nombreux sérovars (au moins 250 sérovars) eux-mêmes regroupés selon leur parenté antigénique en 24 sérogroupes. Une autre classification, dite génomique, basée sur l'homologie génétique telle que l'hybridation ADN/ADN, divise le groupe des leptospires pathogènes en différentes espèces appelé *genomospecies,* telles que *L. interrogans, L. borgpetersenii, L. weilii, L. meyeri, L. kirschneri...*

Ainsi, chez l'homme, les manifestations cliniques peuvent aller de la simple forme grippale (fièvre, céphalées, myalgies) aux atteintes hépatiques et rénales, essentiellement induites par le sérogroupe ICTEROHAEMORRHAGIAE de *Leptospira interrogans.*

Chez le chien, la maladie se traduit par des troubles hépatiques et rénaux fréquemment mortels en l'absence d'antibiothérapie. La vaccination du chien est possible contre 2 sérovars uniquement : Icterohaemorrhagiae et Canicola. Les autres animaux, tels que les ruminants ou les porcs, développent essentiellement des formes chroniques, se traduisant par des troubles de la reproduction, tandis que les chevaux présentent des pathologies diverses associées à des uvéites. Chez les rongeurs, réservoirs de la bactérie, la leptospirose est inapparente.

Compte tenu de l'importance et de la diversité des formes pathologiques, toute suspicion de leptospirose, qu'il s'agisse d'un cas clinique individuel ou d'un problème d'élevage, fait généralement l'objet de méthodes de confirmation expérimentale. Deux volets du diagnostic (et dépistage) peuvent ainsi être nécessaires, en fonction de la durée d'évolution de la maladie. Dans les cas aigus, la mise en évidence directe du germe est primordiale. Dans les cas chroniques, la mise en évidence de façon indirecte par la réponse sérologique est généralement suffisante en terme de diagnostic, même si dans un deuxième temps, la reconnaissance des animaux porteurs de germes, et donc excréteurs, nécessite la mise en évidence du germe.

Le diagnostic sérologique consiste à mettre en contact des antigènes représentés par des souches avec le sérum testé, afin de mettre en évidence les anticorps témoins d'une infection leptospirosique. Le test de Microagglutination (MAT) est actuellement la méthode de référence. Cette technique hautement sensible et spécifique nécessite néanmoins une grande technicité, notamment pour la lecture des résultats et requiert le maintien régulier des cultures de leptospires qui sont souvent exposées au risque de contaminations. De plus, les tests sérologiques ne peuvent pas fournir un diagnostic précoce, car les anticorps n'apparaissent qu'au bout de sept jours après l'apparition des symptômes.

Le diagnostic bactériologique, quant à lui, repose sur l'isolement bactérien à partir du sang, au cours des cinq à sept premiers jours, et à partir du liquide-céphalo-rachidien et des urines après le 12ième jour suivant le début des symptômes. Cependant, l'isolement bactérien nécessite un délai de 10 jours à 2 mois avec une probabilité d'isolement du germe très faible, ce qui limite son intérêt particulièrement dans les formes graves.

La méthode de la réaction en chaîne par polymérase (PCR pour « Polymerase Chain Réaction »), qui permet de détecter l'ADN des microorganismes présents dans les prélèvements, a été beaucoup rapportée du fait de sa sensibilité, spécificité et rapidité en diagnostic bactériologique. Le récent séquençage du génome des leptospires a marqué le début de l'ère post-génomique dans le diagnostic moléculaire de la leptospirose basé sur la PCR. A cet effet, différents outils moléculaires ont été développés : Ils sont basés sur l'amplification d'un ou de plusieurs gènes cibles universels telle que *16*S, *ligA, rrs, gyrB, lipL32* et *secY.* Toutefois, ces systèmes de PCR, classiques pour la plupart, ou de PCR en temps réel utilisant la fluorescence SYBR green présentent des limites de faible spécificité et sensibilité. En outre, la PCR en temps réel requiert une phase de courbe de fusion, afin de vérifier les températures de fusion des amplicons. De plus ils n'ont pas été concrètement validés sur les échantillons biologiques.

Le document WO 99/424478 décrit une protéine de la membrane externe de 32 kDa, dénommée LipL32, exprimée spécifiquement chez les leptospires pathogènes. Il est proposé un kit de diagnostic contenant un acide nucléique codant LipL32 ou des anticorps capables de se lier à LipL32.

Ultérieurement, ce gène a été proposé comme cible pour la mise en oeuvre de la technique dite de PCR en temps réel, dans un test diagnostic *in vitro* (Stoddard et al., Diagnostic Microbiology and Infectious Disease 64 :3 (2009) 247-255). Toutefois, les résultats révèlent une réponse négative dans ce test pour des souches de pathogénicité intermédiaire.

Il existe donc un besoin persistant de développer de nouveaux outils permettant le diagnostic fiable et rapide des souches pathogènes de Leptospires.

### DESCRIPTION DE L'INVENTION

C'est dans ce cadre que s'est inscrite la démarche du présent Demandeur.

La présente divulgation se distingue des solutions de l'art antérieur, notamment basées sur lipL32, puisqu'elle repose sur un gène appelé « gène de ménage », présent chez toutes les souches de leptospires. Il s'agit en l'occurrence du gène *rpoB* qui code la sous-unité β de l'ARN polymérase.

A titre d'exemple, le gène *rpoB* de *Leptospira interrogans* serovar Copenhageni (genre : *Leptospira*; espèce : *interrogans;* sérogroupe : *Icterohaerraorrhagiae ;* sérovar : *Copenhageni*) est situé aux positions 912196 à 915876 du génome et présente la séquence SEQ ID NO : 29.

En pratique, le Demandeur a mis en évidence la présence d'une région de ce gène spécifique des espèces pathogènes.

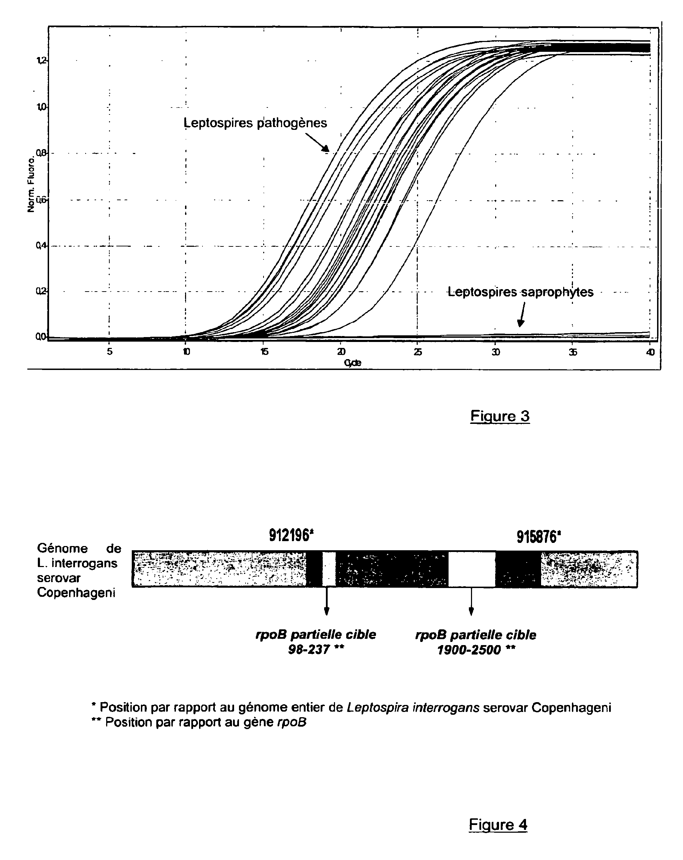

A noter que le gène *rpoB* a déjà été décrit en rapport avec les leptospires mais pour établir un arbre phylogénique et sur la base d'une autre région de 600 pb correspondant aux positions 1900 à 2500, par exemple de la séquence SEQ ID NO : 29 (La Scola et al., FEMS Microbiol. Lett. 263 (2006) 142-147) (Fig. 4). Cette région se distingue de celle mise en évidence dans la présente invention par le fait qu'elle se retrouve de manière conservée aussi bien dans les souches pathogènes que non pathogènes. Leur discrimination nécessite donc le séquençage systématique de cette région.

A noter, en outre, que le fait que cette publication indique une divergence de séquences au niveau d'une région du gène *rpoB* entre souches pathogènes et non pathogènes n'implique nullement que ce gène soit une cible prometteuse pour la discrimination entre ces deux types de souches. En effet et *a contrario,* un autre gène de ménage 16S rna (ou rrs), qui est connu pour sa très grande conservation entre espèces, a été proposé pour détecter spécifiquement les souches pathogènes de leptospires dans un test RT-PCR (Smythe et al., BMC Infectious Diseases, Vol. 2 :13, 2002-07-08).

Ainsi et selon un aspect, la présente divulgation concerne l'utilisation du gène *rpoB* pour diagnostiquer une souche pathogène de leptospire, avantageusement *in vitro.*

En d'autres termes, elle concerne l'utilisation d'un fragment du gène *rpoB* pour détecter spécifiquement *in vitro* une souche pathogène de leptospire.

Plus précisément, ledit fragment correspond à la région 98-237 du gène *rpoB,* notamment celui de la souche pathogène *Leptospira interrogans* serovar Copenhageni qui sert ici de référence. Le terme « correspondant » s'entend comme il sera défini ci-après.

Ainsi et pour la première fois, il a été mis en évidence, dans le gène *rpoB* des leptospires, une région spécifique des souches pathogènes, ayant servi à l'élaboration d'un test diagnostic *in vitro,* très fiable, qui permet en une étape, de manière binaire et sans séquençage, de conclure quant à la présence d'au moins une souche pathogène dans un échantillon testé.

Selon un aspect, la présente invention concerne une méthode pour détecter la présence d'au moins une souche pathogène de leptospire dans un échantillon biologique comprenant l'analyse de l'échantillon biologique pour une séquence correspondant à la séquence de la région 98-237 du gène *rpoB* de *Leptospira interrogans* serovar Copenhageni. La détection de ladite séquence indique la présence d'au moins une souche de leptospire pathogène dans l'échantillon. L'échantillon biologique est analysé par la technique de PCR en temps réel, à l'aide des séquences SEQ ID NO: 25, SEQ ID NO: 26 et SEq ID NO: 27.

En pratique, cette région de 140 pb présente la séquence SEQ ID NO : 2 chez *Leptospira interrogans* serovar Copenhageni.

La séquence du gène *rpoB* pouvant être repérée sur les différents génomes des leptospires, la région correspondante est également aisément localisable.

L'expression « séquence correspondant à la séquence de la région 98-237 du gène *rpoB* de *Leptospira interrogans* serovar Copenhageni » indique que la séquence n'est pas nécessairement strictement identique à ladite séquence SEQ ID NO : 2.

Ainsi, il a pu être mis en évidence dans le cadre de l'invention que cette région était hautement conservée parmi les sérovars connus de leptospires pathogènes avec une identité au moins supérieure à 85%, avantageusement 90, voire 95%, voire 98%, voire même 99% d'identité avec la séquence SEQ ID NO : 2.

En pratique, la séquence correspondante a pu être établie pour 24 de ces sérovars et correspond aux séquences référencées SEQ ID NOS : 1 à 24.

Au contraire, il a été montré dans le cadre de la présente invention que la région correspondante dans des leptospires non pathogènes (par exemple SEQ ID NO : 28) étaient beaucoup plus divergentes, avec un pourcentage d'identité inférieur à 75%.

L'expression « séquence correspondant à la séquence de la région 98-237 du gène *rpoB* de *Leptospira interrogans* serovar Copenhageni » indique également que l'échantillon biologique peut être investiguée pour une séquence de taille un peu différente, tout en permettant encore de discriminer les leptospires pathogènes des non pathogènes.

Ainsi, il s'avère notamment que l'ensemble des séquences SEQ ID NOS : 1 à 24 présentent en amont et en aval des séquences de très forte identité : en amont avec la séquence SEQ ID NO: 25 de 18 pb et en aval avec la séquence inverse complémentaire de SEQ ID NO : 26 de 22 pb. Par conséquent, la région comprenant en outre ces 40 pb peut également être utilisée dans une méthode selon l'invention. La séquence totale présente alors une taille de 180 pb particulièrement bien adaptée pour la détection par PCR, notamment en temps réel.

De manière connue, l'échantillon biologique peut aussi bien être du sang, des urines, du liquide céphalo-rachidien, de l'humeur aqueuse, du sérum, que des organes tels que le rein et le foie. De manière avantageuse, le procédé est mis en oeuvre sur un échantillon de sang ou d'urine.

Par ailleurs, outre un échantillon biologique provenant d'un organisme vivant, avantageusement l'homme ou l'animal, l'échantillon biologique peut provenir de l'environnement. Il peut notamment s'agir d'eaux ou d'échantillons de l'environnement aquatique (boues, ...).

L'analyse de l'échantillon biologique pour la présence de la séquence telle que définie est réalisée par la technique de PCR en temps réel. Cette technique bien connue de l'homme du métier.

Alternativement, il est envisageable de faire cette analyse par hybridation. Ainsi, il est possible de désigner une sonde s'hybridant à la région définie ci-dessus mais de manière spécifique vis-à-vis des leptopsires pathogènes. Une telle sonde peut par exemple avoir une séquence choisie parmi les séquences SEQ ID NO : 1 à 24 ou un fragment de celle-ci ne s'hybridant pas avec la SEQ ID NO : 28 dans les conditions expérimentales retenues.

Cette analyse peut également être réalisée par séquençage directe de cette région.

Le mode de réalisation selon l'invention concerne l'analyse de l'échantillon biologique par la technique de PCR en temps réel à l'aide des séquences SEQ ID NO : 25 et SEQ ID NO : 26 et SEQ ID NO: 27. Là encore, les amorces pouvant être utilisées dans ce cadre ne sont pas strictement limitées aux séquences SEQ ID NOS : 25 et 26 mais couvrent leurs dérivés fonctionnels c'est-à-dire des séquences de taille différente, homologues mais non identiques, mais qui permettent d'obtenir une amplification par PCR uniquement dans les cas de leptospires pathogènes.

Selon l'invention l'analyse de l'échantillon biologique est réalisée par la technique de PCR en temps réel, avec en plus la mise en oeuvre d'une sonde marquée à l'aide de fluorophores. Ladite sonde présente avantageusement la séquence SEQ ID NO : 27.

Selon un autre aspect, l'invention vise également un kit pour détecter la présence d'au moins une souche pathogène de leptospire dans un échantillon biologique contenant au moins trois oligonucléotides comprenant :
- une séquence d'ADN de séquence SEQ ID NO : 25 et SEQ ID NO : 26 et SEQ ID NO : 27 ; ou
- une séquence d'ADN complémentaire à ces séquences ; ou
- une séquence d'ARN correspondant à SEQ ID NO : 25 et SEQ ID NO : 26 et SEQ ID NO : 27 ; ou
- une séquence d'ARN complémentaire à ces séquences.

Dans un mode de réalisation privilégié, et notamment dans le cadre de diagnostics réalisés par PCR, voire par PCR en temps réel, un tel kit comprend des oligonucléotides de séquence SEQ ID NO : 25 et SEQ ID NO : 26, et SEQ ID NO : 27.

L'invention va maintenant être listée de manière non limitative par les exemples suivants, à l'appui des figures annexées.

### LEGENDES DES FIGURES

La figure 1 représente l'analyse par Blast (« Basic Local Alignment Search Tool ») des amorces *rpoB* désignées.
La figure 2 représente l'analyse par Blast (« Basic Local Alignment Search Tool ») de la sonde *rpoB* désignée.
La figure 3 illustre l'amplification par PCR en temps réel duplex du gène partiel *rpoB* des 24 souches de leptospires pathogènes et des 2 souches saprophytes.
La figure 4 schématise le génome de *L. interrogans* serovar Copenhageni et positionne la région d'intérêt pour le test diagnostic selon l'invention.
La figure 5 illustre l'amplification par PCR en temps réel duplex du gène partiel *rpoB* de deux isolats de leptospires pathogènes et de 11 isolats bactériens.
La figure 6 illustre l'amplification par PCR en temps réel duplex du gène partiel *rpoB* des dilutions en série allant de 1x10⁷ à 1x10¹ bactérie/mL de la souche *L.*
*interrogans* serovar Icterohaemrrhagiae.

### EXEMPLES DE REALISATION

### Matériels et méthode

### 1/ Conception des amorces et de la sonde :

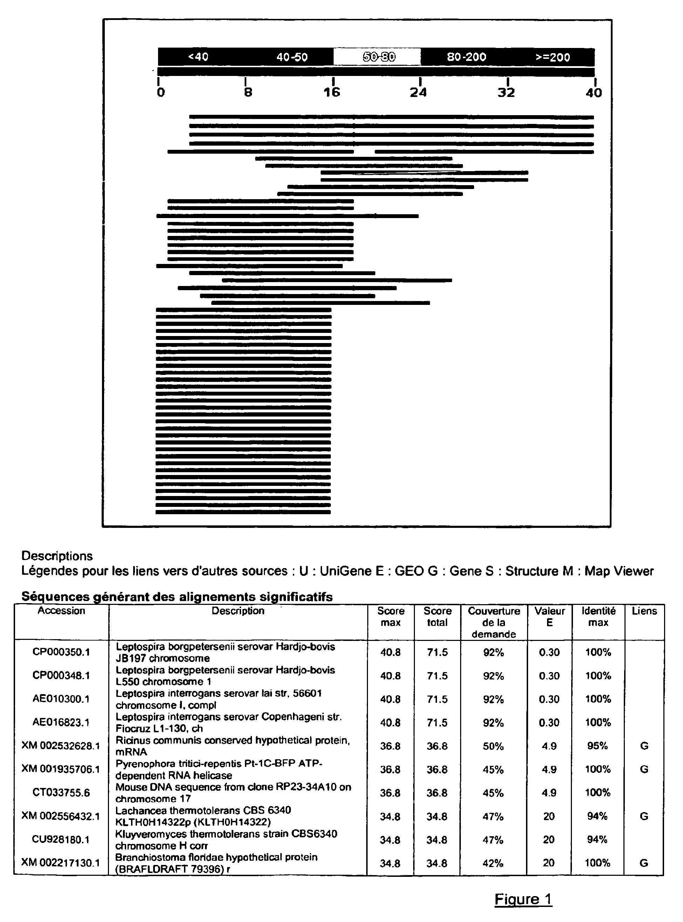

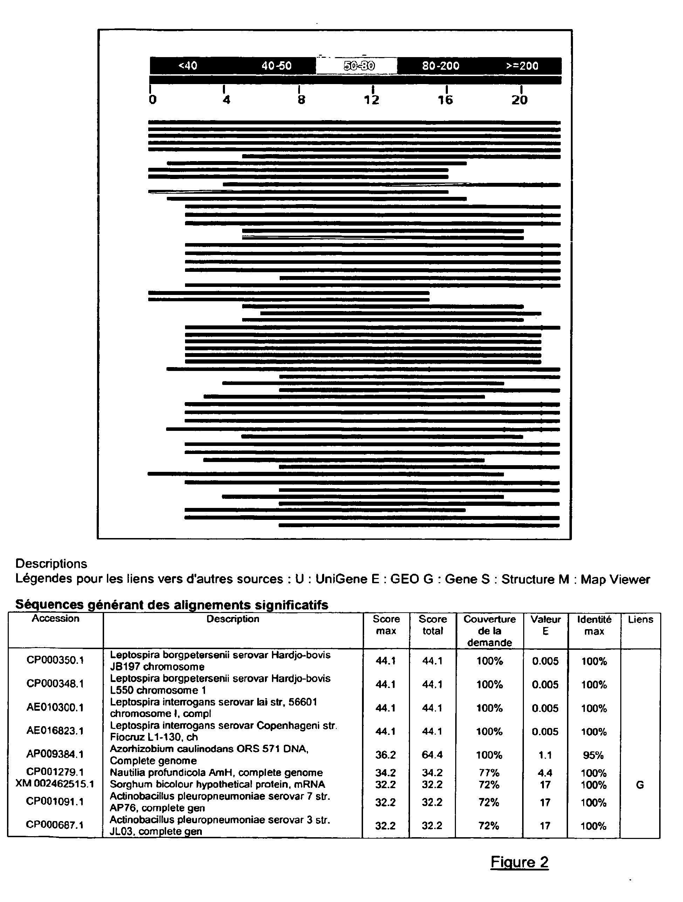

Il a été procédé à une analyse bioinformatique par blast (« Basic Local Alignment Search Tool ») des séquences du gène *rpoB* disponibles sur la database NCBI pour les souches *L. interrogans* (souche pathogène), *L. borgpetersenii* (souche pathogène) et *L. biflexa* (souche non pathogène). Une région d'environ 150 paires de bases (pb), communes aux deux espèces de leptospires pathogènes, a été sélectionnée. Des amorces et une sonde spécifique ont été désignées en utilisant le programme Primer3. La spécificité des amorces (Figure 1) et de la sonde (Figure 2) a été vérifiée *in silico* par blast contre la base de donnée microbes sur NCBI (http://blast.ncbi.nlm.nih.gov/Blast.cgi).

### 2/ Souches bactérienne et prélèvements cliniques :

L'essai de PCR en temps réel a été réalisé sur les 24 souches de références de leptospires pathogènes disponibles au laboratoire, en plus des deux souches de leptospires saprophytes (*L. biflexa* serovar Patoc et *L. meyeri* serovar Semaranga) provenant de l'Unité de Biologie des Spirochètes de l'Institut Pasteur, Paris.

L'ensemble de ces souches est listé dans le Tableau 1 ci-dessous :

**Tableau 1 : Souches de leptospires pathogènes et saprophytes inclues dans l'étude**

| **Sérogroupe** | **Sérovar** | **Séquence de la région *rpoB* associée** |
|---|---|---|
| ICTEROHAEMORRHAGIAE | Icterohaemorrhagiae | SEQ ID NO : 1 |
| | Copenhageni | SEQ ID NO : 2 |
| AUSTRALIS | Muenchen | SEQ ID NO : 3 |
| | Australis | SEQ ID NO : 4 |
| | Bratislava | SEQ ID NO : 5 |
| AUTUMNALIS | Autumnalis | SEQ ID NO : 6 |
| | Bim | SEQ ID NO : 7 |
| BALLUM | Castellonis | SEQ ID NO : 8 |
| BATAVIAE | Bataviae | SEQ ID NO : 9 |
| CANICOLA | Canicola | SEQ ID NO : 10 |
| GRIPPOTYPHOSA | Grippotyphosa | SEQ ID NO : 11 |
| | Vanderhoedoni | SEQ ID NO : 12 |
| HEBDOMADIS | Kremastos | SEQ ID NO : 13 |
| PANAMA | Panama cz214k | SEQ ID NO : 14 |
| | Panama 374 | SEQ ID NO : 15 |
| | Mangus | SEQ ID NO : 16 |
| POMONA | Pomona | SEQ ID NO : 17 |
| PYROGENES | Pyrogenes | SEQ ID NO : 24 |
| SEJROE | Sejroe | SEQ ID NO : 18 |
| | Saxkoebing | SEQ ID NO : 19 |
| | Wolffi | SEQ ID NO : 20 |
| | Hardjo | SEQ ID NO : 21 |
| TARASSOVI | Tarassovi | SEQ ID NO : 22 |
| CYNOPTERI | Cynopteri | SEQ ID NO : 23 |
| BIFLEXA (non pathogène) | Patoc | SEQ ID NO : 28 |
| MEYERI (non pathogène) | Semaranga | |

L'essai a ensuite été validé sur 32 prélèvements cliniques parmi lesquels :
- 17 prélèvements de sang ;
- 11 prélèvements d'urine ; et
- 4 prélèvements d'organes (reins).

### 3/ Extraction d'ADN bactérien :

A partir des souches de leptospires, un volume de 300 µl de culture sur EMJH a été prélevé pour servir à l'extraction d'ADN à l'aide du kit QIAamp DNA kit (QIAGEN, Courtaboeuf, France) selon les instructions du fabricant. L'extraction d'ADN à partir de certains prélèvements cliniques est précédée d'un traitement des échantillons suivant leur nature. En effet pour les organes tels que le foie, rein ou coeur, des tissus de 25 mg sont découpés en petits morceaux, transférés dans un sachet filtre (Filter bags, Seward, Angleterre), puis lacérés et broyés en utilisant le stomacher (Stomacher 400 Circulator, Seward, Angleterre). L'extraction d'ADN est ensuite réalisée sur le broyat obtenu. Les prélèvements d'urine et d'eau sont centrifugés pendant 10 minutes et 30 minutes à 7500 rpm respectivement. Le culot obtenu sert à l'extraction d'ADN.

### 4/ Définition de la spécificité de l'essai:

Afin de tester la spécificité de l'essai, parallèlement au test *in silico* décrit ci-dessus du blast des amorces et de la sonde, l'expérience de PCR en temps réel a été réalisée en présence d'une collection de souches microbiennes, et plus précisément sur les 11 isolats bactériens suivants :
- *Pasteurella aerogenes :*
- *Listeria monocytogenes :*
- *Salmonella enterica* serovar Typhi ;
- *Enterococcus faeculis ;*
- *Escherichia coli ;*
- *Mycobacterium avium silvaticum ;*
- *Pseumonas aeruginosa* ;
- *Legionella pneumophila ;*
- *Actinomyces pyogenes ;*
- *Burkholderia pseudomallei ;* et
- *Shigella boydii.*

Ces microorganismes sont susceptibles d'induire un tableau clinique similaire à la leptospirose ou complique le diagnostic de laboratoire par leur seule présence dans les échantillons et donc de donner de faux positifs dans certains tests diagnostics.

### 5/ Définition de la sensibilité de l'essai :

La sensibilité de l'essai a été testée en utilisant une suspension bactérienne de la souche *L. interrogans* serovar Icterohaemorrhagiae. La suspension a été quantifiée par comptage bactérien (Numération en cellule de Petroff-Hauser) et le nombre de bactéries par millilitre a été calculé. Des dilutions en série ont ensuite été réalisées, allant de 1x10⁷ à 1x10¹ bactéries/mL. Par ailleurs, la répétabilité et la reproductivité de l'essai ont été évaluées sur 3 reliquats pour les différentes dilutions.

### 6/ Réaction d'amplification en temps réel:

Les ADN extraits ont été amplifiés en présence des amorces et la sonde spécifiques ciblant la zone partielle du gène *rpoB,* dont les séquences sont les suivantes :
- une amorce « forward » de 18 pb : rpoB F 5'GTGTTGCCTTCCGAGTCG 3' (SEQ ID NO : 25) ;
- une amorce « reverse » de 22 pb : rpoB R 5'TCTGCAAGAGTTRTTGACCATC3' (SEQ ID NO : 26). A noter que la base dégénérée R est susceptible de reconnaître à la fois la base A et G ;
- une sonde de 22 pb : Fam-5' CGCACGTCCGAGCATATCGTCG 3'-Tamra (SEQ ID NO : 27), Fam et Tamra étant deux marqueurs de fluorescence.

Le mélange réactionnel (mix) pour chaque échantillon est composé de :
- 12.5 µL de « universal PCR Master mix 2x » (Applied Biosystems, Courtaboeuf, France) ;
- 2.5 µL de « exogenous internal positive control mix 10x » (Applied Biosystems) ;
- 0.5 µL de « exogenous internal positive control DNA » ;
- 200 nmol/L de chaque amorce ;
- 100 nmol/L de sonde ; et
- 1 µL d'ADN cible
pour un volume final de 25µl ajusté avec de l'eau distillée stérile.

La réaction de PCR en temps réel comprend une étape d'activation à 95°C pendant 10 minutes, suivie par 40 cycles d'amplification à 95°C pendant 15 secondes et 60°C pendant 1 minute. Un contrôle positif interne est utilisé comme contrôle, d'où l'appellation « PCR en temps réel en duplex de l'essai », et ceci pour évaluer les inhibiteurs de la PCR en présence de certains prélèvements biologiques, tels que les urines susceptibles de contenir beaucoup d'inhibiteurs. Des contrôles négatifs comprenant le mix sans la matrice sont également inclus dans la réaction.

### 7/ Diagnostic de référence :

Afin de valider les résultats sur les prélèvements cliniques, il a été procédé, en parallèle à l'essai de PCR en temps réel duplex décrit ci-dessus, une analyse de référence par MAT (Microscopique Agglutination Test) effectué sur le sérum correspondant aux prélèvements cliniques, mais aussi à la mise en culture de ces prélèvements sur milieu Ellinghausen-McCullough-Johnson-Harris (EMJH) incubés à 30°C pendant 10 semaines.

### Résultats

### 1/ Essai de PCR en temps réel:

Une amplification positive a été obtenue en présence des 24 souches de leptospires pathogènes. En revanche, aucun signal n'a été détecté avec les souches saprophytes ainsi que pour les contrôles négatifs (Figure 3). Ceci permet de conclure que le test est 100% spécifique des leptospires pathogènes.

En analysant les séquences amplifiées entre les deux amorces rpoB F (SEQ ID NO : 25) et rpoB R (SEQ ID NO : 26), il a été possible d'obtenir la séquence exacte de cette région dans les différentes sérovars de leptospires pathogènes. Ces séquences, dénommées SEQ ID NO : 1 à 24, sont répertoriées dans le tableau 1 ci-dessus. Elles contiennent de 139 à 141 pb, la grande majorité de ces séquences (22 sur 24) ayant 140 bp.

Toutes ces séquences sont flanquées en amont d'une séquence de 18 pb identique à la séquence de l'amorce rpoB F, à savoir la séquence SEQ ID NO : 25 et en aval d'une séquence de 22 pb correspondant à la séquence complémentaire inverse de l'amorce rpoB R, à savoir la séquence SEQ ID NO : 26. La séquence globale de ces régions a donc une taille d'environ 180 pb.

Par ailleurs, la séquence de la sonde (SEQ ID NO : 27) est retrouvée à 2 bases près dans chacune de ces séquences SEQ ID NOS : 1 à 24, par exemple aux positions 114 à 135 de la SEQ ID NO : 1.

Une comparaison de ces séquences entre elles révèle une grande homogénéité, avec un pourcentage de divergence entre les différents sérovars pathogènes (SEQ ID NOS : 1 à 24) qui varient de 100 à 87,4%.

A titre d'exemple, cette région a pu être localisée sur le génome de *L. interrogans* serovar Copenhageni. Comme illustré à la figure 4, la séquence SEQ ID NO : 2 est localisée aux positions 98 à 237 par rapport au gène *rpoB,* ledit gène correspondant aux positions 912196 à 915876 du génome et présentant la séquence SEQ ID NO : 29.

Par ailleurs, l'utilisation des deux amorces décrites ci-dessus a permis de localiser une région équivalente chez *L. Biflexa* mais qui n'est pas amplifiée dans le cadre du présent test diagnostic : il s'agit de la séquence SEQ ID NO : 28, dont le pourcentage de divergence avec les séquences des leptospires pathogènes est d'au moins 74.29%.

### 2/ Spécificité et sensibilité de l'essai :

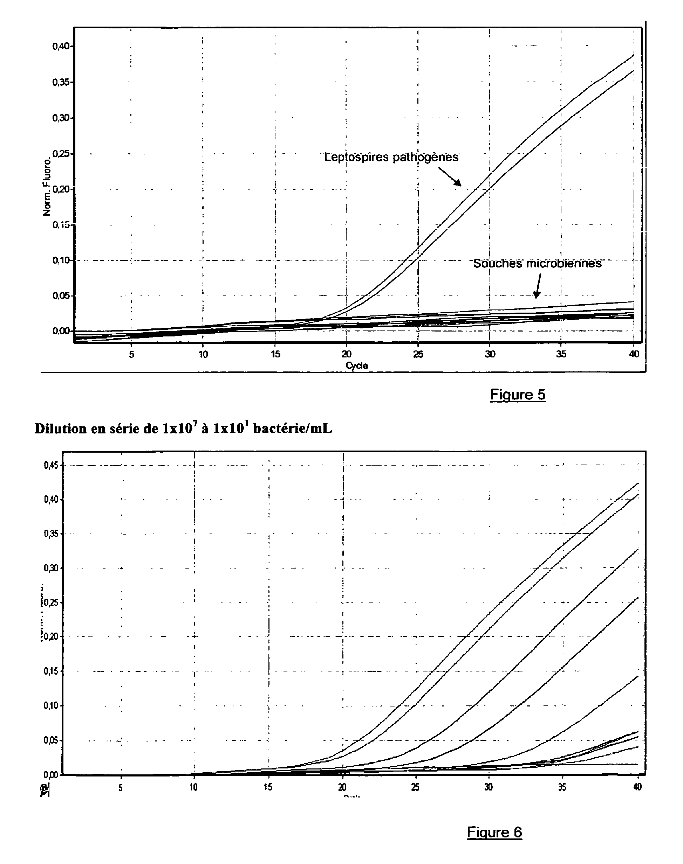

Afin de tester la spécificité du système, le même essai a été mis en oeuvre en présence d'une collection de 11 souches microbiennes. La PCR en temps réel n'a permis de détecter aucun signal d'amplification sur ces isolats bactériens (Figure 5).

Par ailleurs l'essai s'est avéré très sensible puisqu'il est possible de détecter des leptospires pathogènes jusqu'à une dilution minimale de 1x10¹ bactérie/mL (Figure 6).

Enfin, les triplicats d'ADN de souches de leptospires pathogènes, amplifiés en essai de PCR en temps réel dans le même « run » ou dans des « runs » différents, donnent un signal positif avec une variation du nombre de cycles « thresholds » (Ct) allant de 0 à 0.5 Ct.

### 3/ Détection des leptospires pathogènes dans les prélèvements cliniques:

L'essai de PCR en temps réel réalisé sur les prélèvements de sang, d'urine et d'organe a permis de détecter 18 positifs sur 32 échantillons. Ces résultats positifs sont en corrélation avec l'analyse sérologique par MAT et la culture. Les prélèvements révélés négatifs par l'essai sont également négatifs en sérologie et en culture de 10 semaines.

Par ailleurs, concernant la sensibilité de l'essai sur les prélèvements cliniques, l'essai a permis de détecter jusqu'à 1x10¹ bactérie/mL. Toutefois, il a été noté des valeurs de Ct constantes pour les répliquats, mais élevées (avoisinant les 39 cycles), ce qui s'explique probablement par le potentiel inhibiteur que contiennent les prélèvements biologiques d'une façon générale.

Pour les urines, l'effet d'inhibiteurs est plus prononcé puisque les prélèvements d'urine s'effectuent à partir de valeurs de Ct de 40 cycles pour la cible *rpoB,* ce qui justifie la nécessité de centrifuger les urines afin de concentrer les leptospires avant l'extraction d'ADN.

### Conclusions

Il a été mis au point et évalué un essai de PCR en temps réel avec une sonde Taqman ciblant la région partielle du gène *rpoB* pour la détection des leptospires pathogènes.

L'essai s'est révélé parfaitement spécifique puisqu'il permet la détection des leptospires pathogènes, mais pas celle de leptospires saprophytes ou d'autres souches microbiennes susceptibles d'induire des faux positifs.

Par ailleurs, le test est reproductible et permet de détecter jusqu'à 1x10¹ bactérie/mL, aussi bien dans les cultures que dans les prélèvements cliniques, avec des différences de Ct qui varient suivant la nature du prélèvement.

L'essai de PCR en temps réel est un apport important pour le diagnostic précoce de la leptospirose qui remplacerait la sérologie et la culture, particulièrement dans les cas de forme grave de la maladie ou dans les situations d'épidémie au sein d'un élevage animal ou chez l'homme.

## Revendications

1. Méthode pour détecter *in vitro* la présence d'au moins une souche pathogène de leptospire dans un échantillon biologique comprenant l'analyse de l'échantillon biologique pour une séquence présentant au moins 85% d'identité avec la séquence de la région 98-237 (SEQ ID NO : 2) du gène *rpoB* de *Leptospira interrogans* serovar Copenhageni,
la détection de ladite séquence indiquant la présence d'au moins une souche de leptospire pathogène dans l'échantillon,
dans laquelle l'échantillon biologique est analysé par la technique de PCR en temps réel, à l'aide des séquences SEQ ID NO : 25, SEQ ID NO : 26 et SEQ ID NO : 27.

2. Méthode selon la revendication 1 ***caractérisée* en ce que** l'échantillon biologique est analysé pour une séquence présentant au moins 90%, voire 95%, voire 98%, voire même 99% d'identité avec la séquence SEQ ID NO : 2.

3. Méthode selon la revendication 2 ***caractérisée* en ce que** l'échantillon biologique est analysé pour une séquence choisie dans le groupe des séquences SEQ ID NO : 1 à 24.

4. Méthode selon l'une des revendications 1 à 3 ***caractérisée* en ce que** l'échantillon biologique est du sang, des urines, du liquide céphalo-rachidien, de l'humeur aqueuse, du sérum, des organes tels que le rein, avantageusement du sang.

5. Méthode selon l'une des revendications 1 à 3 ***caractérisée* en ce que** l'échantillon biologique est de l'eau ou un échantillon de l'environnement aquatique.

6. Kit pour détecter la présence d'au moins une souche pathogène de leptospire dans un échantillon biologique contenant au moins trois oligonucléotides comprenant respectivement :
- une séquence d'ADN de séquence SEQ ID NO : 25, une séquence d'ADN de séquence SEQ ID NO : 26 et une séquence d'ADN de séquence SEQ ID NO : 27 ; ou
- une séquence d'ADN complémentaire à ces séquences ; ou
- une séquence d'ARN correspondant à SEQ ID NO : 25, SEQ ID NO : 26 et SEQ ID NO : 27 ; ou
- une séquence d'ARN complémentaire à ces séquences.

7. Kit selon la revendication 6 ***caractérisé* en ce qu'**il comprend trois oligonucléotides de séquence SEQ ID NO : 25, SEQ ID NO : 26, et SEQ ID NO : 27, respectivement.

## Claims

1. A method of *in vitro* detection of the presence of at least one pathogenic leptospire strain in a biological sample, comprising the analysis of the biological sample for a sequence having at least a 85% identity with the sequence of region 98-237 (SEQ ID NO: 2) of the *rpoB* gene of *Leptospira interrogans* serovar Copenhageni,
the detection of said sequence indicating the presence of at least one pathogenic leptospire strain in the sample,
wherein the biological sample is analyzed by the real time PCR technique, by means of sequences SEQ ID NO: 25, SEQ ID NO: 26, and SEQ ID NO: 27.

2. The method of claim 1, ***characterized in that*** the biological sample is analyzed for a sequence having an identity of at least 90%, or even 95%, or even 98%, or even up to 99% with sequence SEQ ID NO: 2.

3. The method of claim 2, ***characterized in that*** the biological sample is analyzed for a sequence selected from the group of sequences SEQ ID NO: 1 to 24.

4. The method of any of claims 1 to 3, ***characterized in that*** the biological sample is blood, urine, cerebrospinal fluid, aqueous humor, serum, organs such as the kidney, and advantageously blood.

5. The method of any of claims 1 to 3, ***characterized in that*** the biological sample is water or an aquatic environment sample.

6. A kit for detecting the presence of at least one pathogenic leptospire strain in a biological sample containing at least three oligonucleotides respectively comprising:
- a DNA sequence of sequence SEQ ID NO: 25, a DNA sequence of sequence SEQ ID NO: 26, and a DNA sequence of sequence SEQ ID NO: 27; or
- a DNA sequence complementary to these sequences; or
- an RNA sequence corresponding to SEQ ID NO: 25, SEQ ID NO: 26, and SEQ ID NO: 27; or
- an RNA sequence complementary to these sequences.

7. The kit of claim 6, ***characterized in that*** it comprises three oligonucleotides of sequence SEQ ID NO: 25, SEQ ID NO: 26, and SEQ ID NO: 27, respectively.

## Patentansprüche

1. Verfahren, um *in vitro* das Vorhandensein mindestens eines pathogenen Leptospirenstamms in einer biologischen Probe nachzuweisen, die Analyse der biologischen Probe auf eine Sequenz hin umfassend, die mindestens 85% Übereinstimmung mit der Sequenz des Bereichs 98-237 (SEQ ID Nr. 2) des Gens *rpoB* von *Leptospira interrogans* serovar Copenhageni aufweist,
wobei der Nachweis der Sequenz das Vorhandensein mindestens eines pathogenen Leptospirenstamms in der Probe anzeigt,
wobei die biologische Probe durch das PCR-Verfahren in Echtzeit mittels der Sequenzen SEQ ID Nr. 25, SEQ ID Nr. 26 und SEQ ID Nr. 27 analysiert wird.

2. Verfahren nach Anspruch 1, ***dadurch gekennzeichnet, dass*** die biologische Probe auf eine Sequenz hin analysiert wird, die mindestens 90%, sogar 95%, sogar 98%, sogar 99% Übereinstimmung mit der Sequenz SEQ ID Nr. 2 aufweist.

3. Verfahren nach Anspruch 1, ***dadurch gekennzeichnet, dass*** die biologische Probe auf eine Sequenz hin analysiert wird, die aus der Gruppe der Sequenzen SEQ ID Nr. 1 bis 24 ausgewählt ist.

4. Verfahren nach einem der Ansprüche 1 bis 3, ***dadurch gekennzeichnet, dass*** die biologische Probe Blut, Urin, Gehirn-Rückenmarksflüssigkeit, Kammerwasser, Serum, Organe wie etwa die Niere, vorteilhafter Weise Blut ist.

5. Verfahren nach einem der Ansprüche 1 bis 3, ***dadurch gekennzeichnet, dass*** die biologische Probe Wasser oder eine Probe der Gewässerumgebung ist.

6. Kit zum Nachweisen des Vorhandenseins mindestens eines pathogenen Leptospirenstamms in einer biologischen Probe, die mindestens drei Oligonukleotide enthält, die jeweils umfassen:
- eine DNA-Sequenz mit der Sequenz SEQ ID Nr. 25, eine DNA-Sequenz mit der Sequenz SEQ ID Nr. 26 und eine DNA-Sequenz mit der Sequenz SEQ ID Nr. 27; oder
- eine zu diesen Sequenzen komplementäre DNA-Sequenz; oder
- eine RNA-Sequenz, die SEQ ID Nr. 25, SEQ ID Nr. 26 und SEQ ID Nr. 27 entspricht; oder
- eine zu diesen Sequenzen komplementäre RNA-Sequenz.

7. Kit nach Anspruch 6, ***dadurch gekennzeichnet, dass*** es drei Oligonukleotide mit der Sequenz SEQ ID Nr. 25, SEQ ID Nr. 26 bzw. SEQ ID Nr. 27 umfasst.


### SEQUENCE LISTING

<110> INSTITUT D'ENSEIGNEMENT SUPERIEUR ET DE RECHERCHE EN ALIMENTATION, SANTE ANIMALE, SCIENCES AGRONOMIQUES ET DE L'ENVIRONNEMENT
<120> METHODE DE DETECTION DES LEPTOSPIRES PATHOGENES BASEE SUR LE GENE RPOB
<130> E131-B-31102 EP
<150> FR09.58895
   <151> 2009-12-11
<160> 29
<170> PatentIn version 3.3
<210> 1
   <211> 140
   <212> DNA
   <213> Icterohaemorrhagiae Icterohaemorrhagiae
<400> 1
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 2
   <211> 140
   <212> DNA
   <213> Icterohaemorrhagiae Copenhageni.
<400> 2
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 3
   <211> 140
   <212> DNA
   <213> Australis Muenchen
<400> 3
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 4
   <211> 140
   <212> DNA
   <213> Australis Australis
<400> 4
   gtaataataa tatccagagc aagtcctcta agctcttgaa ccaatacgtt gaaggattcg 60
   gggataccag gttttataga atggatccct tttacgatcg cctcgtagat tctcgcacgt 120
   ccgagcatat cgtcggactt 140
<210> 5
   <211> 140
   <212> DNA
   <213> Australis Bratislava
<400> 5
   gtaataataa tatccagagc aagtcctcta agctcttgaa ccaatacgtt gaaggattcg     60
   gggataccag gttttataga atggatccct tttacgatcg cttcgtagat tctcgcacgt    120
   ccgagcatat cgtcggactt                                                140
<210> 6
   <211> 140
   <212> DNA
   <213> Autumnalis Autumnalis
<400> 6
   gtaataataa tatccagagc aagtcctcta agctcttgaa ccaatacgtt gaaggattcg     60
   gggataccag gttttataga atggatccct tttacgatcg cttcgtagat tctcgcacgt    120
   ccgagcatat cgtcggactt                                                140
<210> 7
   <211> 140
   <212> DNA
   <213> Autumnalis Bim
<400> 7
   gtgatgataa tatccaaagc aagtcctcta agctcttgaa ccaatacgtt gaaggattca     60
   ggaatacccg gtttgatcga gtgaattccc tttacgatcg cttcgtagat tctcgcacgt    120
   ccgagcatat cgtcggactt                                                140
<210> 8
   <211> 140
   <212> DNA
   <213> Ballum Catsellonis
<400> 8
   gtgatgataa tatccagagc aagtcctctc agctcttgaa ccaatacgtt gaaggattca     60
   gggatacccg gtttgatcga gtgaattccc tttacgatcg cttcgtagat tctcgcgcgt    120
   ccgagcatat catcggactt                                                140
<210> 9
   <211> 140
   <212> DNA
   <213> Bataviae bataviae
<400> 9
   gtaataataa tatccagagc aagtcctcta agctcttgaa ccaatacgtt gaaggattcg 60
   gggataccag gttttataga atggatccct tttacgatcg cctcgtagat tctcgcacgt 120
   ccgagcatat cgtcggactt 140
<210> 10
   <211> 140
   <212> DNA
   <213> Canicola Canicola
<400> 10
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 11
   <211> 140
   <212> DNA
   <213> Grippotyphosa Grippotyphosa
<400> 11
   gtgatgataa tatccaaagc aagtcctcta agctcttgaa ccaatacgtt gaaggattca 60
   ggaatacccg gtttgatcga gtgaattccc tttacgatcg cttcgtagat tctcgcacgt 120
   ccgagcatat cgtcggactt 140
<210> 12
   <211> 140
   <212> DNA
   <213> Grippotyphosa Vanderhoedoni
<400> 12
   gtgatgataa tatccaaagc aagtcctcta agctcttgaa ccaatacgtt gaaggattca 60
   ggaatacccg gtttgatcga gtgaattccc tttacgatcg cttcgtagat tctcgcacgt 120
   ccgagcatat cgtcggactt 140
<210> 13
   <211> 140
   <212> DNA
   <213> Hebdomadis Kremastos
<400> 13
   gtaataataa tatccagagc aagtcctcta agctcttgaa ccaatacgtt gaaggattcg 60
   gggataccag gcttgataga atgaattccc tttacgatcg cttcgtagat tctcgcacgt 120
   ccgagcatat cgtcggactt 140
<210> 14
   <211> 141
   <212> DNA
   <213> Panama Panama cz214k
<400> 14
   gtgatgataa tatccagagc aagtcctctc agctcttgaa ccaatacgtt gaaggattca 60
   gggataccca ggtttgatcg agtgaattcc ctttacgatc gcttcgtaga ttctcgcgcg 120
   tccgagcata tcatcggact t 141
<210> 15
   <211> 139
   <212> DNA
   <213> Panama Panama 374
<400> 15
   gtaataataa tatccagagc aagtcctcta agctcttgaa ccaatacgtt gaaggattcg 60
   gggataccag gcttgataga atgattccct ttacgatcgc ttcgtagatt ctcgcacgtc 120
   cgagcatatc ttcggactt 139
<210> 16
   <211> 140
   <212> DNA
   <213> Panama Mangus
<400> 16
   gtgatgataa tatccagagc aagtcctcta agctcttgaa ccaatacgtt gaaggattcg 60
   gggataccag gtttgatgga atggattccc tttacgatcg cttcgtagat tctcgcgcgt 120
   ccgagcatat cgtcggactt 140
<210> 17
   <211> 140
   <212> DNA
   <213> Pomona Pomona
<400> 17
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 18
   <211> 140
   <212> DNA
   <213> Sejroe Sejroe
<400> 18
   gtgatgataa tatccagagc aagtcctctc agctcttgaa ccaatacgtt gaaggattca 60
   gggatacccg gtttgatcga gtgaattccc tttacgatcg cttcgtagat tctcgcgcgt 120
   ccgagcatat catcggactt 140
<210> 19
   <211> 140
   <212> DNA
   <213> Sejroe Saxkoebing
<400> 19
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 20
   <211> 140
   <212> DNA
   <213> Sejroe wolffi
<400> 20
   gtaataataa tatccagagc aagtcctcta agctcttgaa ccaatacgtt gaaggattcg 60
   gggataccag gttttataga atggatccct tttacgatcg cctcgtagat tctcgcacgt 120
   ccgagcatat cgtcggactt 140
<210> 21
   <211> 140
   <212> DNA
   <213> Sejroe Hardjo
<400> 21
   gtaataataa tatccagagc aagtcctcta agctcttgaa ccaatacgtt gaaggattcg 60
   gggataccag gttttataga atggatccct tttacgatcg cttcgtagat tctcgcacgt 120
   ccgagcatat cgtcggactt 140
<210> 22
   <211> 140
   <212> DNA
   <213> Tarassovi Tarassovi
<400> 22
   gtgatgatga tatccagagc aagtcctctc agctcttgaa ccaatacgtt gaaggattca 60
   gggatacccg gtttgatcga gtgaattccc tttacgatcg cttcgtagat tctcgcacgt 120
   ccgagcatat cgtcggactt 140
<210> 23
   <211> 140
   <212> DNA
   <213> Cynopteri Cynopteri
<400> 23
   gtgatgataa tatccaaagc aagtcctcta agctcttgaa ccaatacgtt gaaggattca 60
   ggaatacccg gtttgatcga gtgaattccc tttacgatcg cttcgtagat tctcgcacgt 120
   ccgagcatat cgtcggactt 140
<210> 24
   <211> 140
   <212> DNA
   <213> Pyrogenes Pyrogenes
<400> 24
   gtgatgataa tatccaaagc aagtcctcta agctcttgaa ccaatacgtt gaaggattca 60
   ggaatacccg gtttgatcga gtgaattccc tttacgatcg cttcgtagat tctcgcacgt 120
   ccgagcatat cgtcggactt 140
<210> 25
   <211> 18
   <212> DNA
   <213> Artificial sequence
<220>
   <223> rpoB F
<400> 25
   gtgttgcctt ccgagtcg 18
<210> 26
   <211> 22
   <212> DNA
   <213> Artificial sequence
<220>
   <223> rpoB R
<400> 26
   tctgcaagag ttrttgacca tc 22
<210> 27
   <211> 22
   <212> DNA
   <213> Artificial sequence
<220>
   <223> Sonde *rpoB*
<400> 27
   cgcacgtccg agcatatcgt cg 22
<210> 28
   <211> 137
   <212> DNA
   <213> Biflexa Patoc
<400> 28
   atgatgatat caagtgcgag acctcggagt tcttgtacaa gaacgttgaa ggattcaggg    60
   atacccggtt tgatcgagtg gatccctttc acaattgctt cgtaaattct ggcacgtccg   120
   agcatgtcat ctgactt                                                  137
<210> 29
   <211> 3681
   <212> DNA
   <213> Leptospira *interrogans* serovar Copenhageni
<400> 29
   ttacgcgttc tctatagtct cgaatttaat tttcttctta ctcttagaat actcatcctc    60
   gtagtcggaa atatcaaccg tgttgccttc cgagtcggta ataataatat ccagagcaag   120
   tcctctaagc tcttgaacca atacgttgaa ggattcgggg ataccaggtt ttatagaatg   180
   gatccctttt acgatcgctt cgtagattct cgcacgtccg agcatatcgt cggacttgat   240
   ggtcaacaac tcttgcagag tgtgggaagc gccgtaagct tcgagagccc agacttccat   300
   ctccccaaga cgctgacccc cgaactgagc ctttcctccg agaggttgtt gagtaaccag   360
   agagtaaggt ccggtcgatc ttgcgtgaat cttgtcttcc accaagtgag cgagttttaa   420
   gatgtagata taaccgcaga aaacttcgtt cataaaagga agtccggttc taccgtcata   480
   caatttgaat ttagaattca acggaagatt tgcctcttta cagaagttgt ccacatcgga   540
   ttcttccgca ccgtcgaaca ccggagtttc aaaagaaatt ccgagtttgc tcgccgcgaa   600
   accgagttgt gtttcgaaaa tctgaccgag gttcatccgt gaaggaacac ctaacgggtt   660
   taagacgatg tccaaaggag ttccgtcttc catgtaaggc atgtcttctt ccgccataac   720
   gcgggcgact acccctttgt ttccgtgacg tccggccatt ttatctccga ccagaagttt   780
   tctctttctg gcaacgaaaa ctttcaccat ttcttcgacg cccgcaggaa gttcgtcttg   840
   gttttcacgg gagaatcttt taatatcgat gacggttcct tcgaaaccgt tcggcattct   900
   taaggaagaa tcacgaacgt ctttcgcttt ctcaccgaag atcgaatgaa gaagtttgta   960
   ttccggtgta agatctgttt cgccttttgg agtcaccatt cccacgagaa tgtctcccgg  1020
   ttttacttcc gcaccgatgc ggattactcc ggtttcatcc aaatcacgga acgctttgtc  1080
   cgaaagattc ggaatatcgc gcgtgatttg ctctggtcca agcttggttt ctcttgcttg 1140
   gatttcgaac tcttcaatgt gaatggaaga gaatacgtcg tcacggacga ttctttcgga 1200
   aattaaaatc gcatcctcga agttgtaacc ttcccaaggc atgaacgccg cgagaacgtt 1260
   tcttcccaga gcgagaactc cgttgtcgac cgcaggacca tcagcaagaa cggttccttt 1320
   tacgaggata tttcccatct catccaactt ctcacctaca acaacttgtc ctgcgagagt 1380
   cgatcctctt tttacttctt cgcctgcaga gacgatcgga gaatattgtt tgcttccgat 1440
   ttgaagaaca tattctttca gttcaccgtt ttcaccagtg acttcgattt tttctttgga 1500
   aaccttggaa acctttccat tgatctcgga gtgaaccact cctacaatcg gcttctgatt 1560
   aaagcaggtt ccttggtttg tctttttgaa tttcgtaagt tgatacgtat cggattcttt 1620
   tccgcccttt ctttctacaa cgatgttttc cgcatcgacg gaagttacga caccgtcgtg 1680
   tttgtttacg atacaaattc tggaatcgta agcggctctg gtttccatac cagttcctac 1740
   aaaaggagct tcttcacgga gaagaggaac cgcctgacgt tgcatgttgg aacccatgag 1800
   ggcgcggttc gcgtcgtcgt gttcaaggaa cggaatcagc gccgtggaaa ccgaaacgac 1860
   ttgtagagga gccaagtcca tatactgaat ctcgctcggg ttacggaaag ggaaatcccc 1920
   tctgtgacgc gtggaaatca atttgttttt gagctcgcct ttttcatcga tcacgccgga 1980
   agcttgagcg atgtaatgat attcttcttt gtctgcggta aggtgctcga tctgaccggt 2040
   aactttaccg ttcttcacgg ttctgtaagg agtttccaag aatccgtagt cattcacacg 2100
   agcgtaagaa gacatggaaa gaatcagacc gatgttcgga ccttccggag tttcaatcgg 2160
   acacattcta ccgtagtgag aatagtgaac gtcacgcact tccatacccg ctctgtctct 2220
   ggaaagacca ccgggaccga gagcgtttaa tcttcgttta tgggtcaact ccgcgagagg 2280
   gtttgtctga tccataaact gagaaagctg agaagaaccg aaaaattcat tgatcactgc 2340
   agtgatcggt ttgatcgaaa tgagaagttg aggagtttgt gtttcgatct cctgaaccgt 2400
   cattctttct ttgattaccc tttccactct ggaaaatccg gtcttgagtt ggttggaaat 2460
   caattctcca acggaacgga tccttctgtt tccaagatga tcaatatcgt ccggataata 2520
   attttctgtt tctgagaaaa gattcagaat gtaacgaacg gtttcaatga tgtcagcagg 2580
   tcttaaaaca cgggactttt caccggagaa ttctttcggg ttgttgaact cgaacttgga 2640
   attgatttta taacgaccca cttctccgag atcgaacgtt tttggagaaa agaaaagacg 2700
   ggtcaattca gtcgtcgcgt tttcgatcgt ggaaggctca ccttgacgca tgagagaatg 2760
   gaacttgagg atcgcatcct catagtcgtt gaccccatct ttttcaagag cattgatcaa 2820
   aatcggattg tcttttcctt ttgggaattc gatcaactct acttctttta ccttcatctc 2880
   ttttaagatg gagatattgt cttcattgat cttggaaccg gcttcgagca ttacctctcc 2940
   ggtttccatg ttgatgatgt cgttgatagt tcttcttccg agaattttct tcagatcttt 3000
   ggaagtggcg cccgcaattt tttctttttt agaactgtaa aacagtcgaa gaacctcttc 3060
   gttcgttcca tgtcctaggg atttaacgag aagggtggca ggaaattttt tctttctatc 3120
   gatctttgcg atcaaaattc ccttgttatc catctcaaat tccaaccaag aacctctgta 3180
   aggaatcact ctggcggaga agacgtccct ttccatatca taagaaaaga atataccagg   3240
   agaacgatga agctgagaaa cgactacacg ttccgctcca ttgatgataa aagtaccttg   3300
   ttcggtcatc acgggaagat ctcccatgta aaccgtttgc tctcggattt ctccggtttc   3360
   tttgatgatc aatcggatca ccgcttttaa cggcatcgca aaagttgcgt ccgtgtcctt   3420
   acattcttga ggagaacgtt tcggttctcc tagaatataa tgactatatt ccatgatcat   3480
   gtcgttattc ggactttcaa tgggaaaggt ttcccgaaat acggcttcta acccttgatg   3540
   tcttctttta gtttcgtctt tgacatctgc ttgaagaaac cagtcaaaag aacgtttttg   3600
   aatttgaatc aagttaggaa ggtaatccag attcgtaatt tttccgaaat ttacccgttt   3660
   tctctctact tgaccgtaca t                                             3681